# EUROPEAN PATENT SPECIFICATION

(11) **EP 4 125 550 B1**
(45) Date of publication and mention of the grant of the patent: **02.07.2025**
(21) Application number: 21711261.4
(22) Date of filing: 15.03.2021
(51) Int. Cl.: A61B 5/00, A61B 5/349, A61N 1/37, A61N 1/368, A61B 5/353, A61B 5/364

(54) **IMPLANTABLE MEDICAL DEVICE FOR DETECTING ATRIAL UNDERSENSING**
IMPLANTIERBARE MEDIZINISCHE VORRICHTUNG ZUR DETEKTION VON VORHOF-UNTERSENSING
DISPOSITIF MÉDICAL IMPLANTABLE POUR DÉTECTER UNE SOUS-DÉTECTION AURICULAIRE

(30) Priority: 30.03.2020 EP 20166663
(43) Date of publication of application: 08.02.2023
(73) Proprietor: BIOTRONIK SE & Co. KG, 12359 Berlin (DE)
(72) Inventor: FISCHER, René, 10245 Berlin (DE); THEILER, Tobias, 10829 Berlin (DE)
(74) Representative: Biotronik Corporate Services SE
(86) International application number: PCT/EP2021/056512
(87) International publication number: WO 2021/197819

(56) References cited:
- EP-A2- 1 302 215
- US-A1- 2013 053 714
- US-B1- 8 583 221
- US-B2- 8 750 976

## Description

The present invention relates to an implantable medical device according to the preamble of claim 1, to a method for recognizing a specific condition of such an implantable medical device according to the preamble of claim 12, and to a computer program product that can be used for controlling such an implantable medical device according to the preamble of claim 13.

During the operation of an implantable medical device for stimulating a human or animal heart, it is necessary to reliably determine the cardiac state of the heart to be eventually stimulated. Generally, the better the detection of a specific cardiac rhythm, the better are the possibilities of treating an abnormal cardiac rhythm. A major concern is the discrimination between ventricular tachycardia (VT) and a supraventricular tachycardia (SVT). For such discrimination, it is necessary to determine the relationship between atrial and ventricular activity of the human or animal heart that is to be eventually stimulated.

Atrial undersensing, i.e. an insufficient sensing or detection of atrial signals, is a generally known problem. Atrial undersensing means that - despite of sufficiently high atrial activity - atrial signals cannot be detected or can be detected only to an insufficient amount. Atrial undersensing can lead to a misinterpretation of atrial activity as lacking atrial activity. In such a case, an inadequate treatment of the respective patient could be triggered, e.g. by an inadequate stimulation of the human or animal heart.

Atrial undersensing can specifically occur in case of floating electrode poles, i.e., an electrode lead being anchored in the ventricle but having two electrode poles located on the lead body at the level of the atrium of the same heart. The two electrodes are therefore 'floating' in the atrium without being fixed to its walls. Such floating electrode poles measure atrial signals via the blood and not directly at the cardiac muscle. Consequently, the signals detected by such floating electrode poles are much smaller than in case of electrodes being anchored within the atrium. Floating electrode poles are particularly prone to dislocation and to sensing of atrial signals with varying intensity.

EP1302215A2 describes an implantable cardiac stimulation device and an associated method to perform a true or blanking period ventricular undersensing detection algorithm in response to ventricular loss of capture not associated with fusion or a change in capture threshold.

US8583221B1 discloses a medical device system which senses cardiac signals and generates and stores sensing data including sensed cardiac events. A processor receiving the sensing data is configured to detect undersensed and oversensed events.

US8750976B2 teaches an implantable medical device which acquires a first cardiac signal in a first heart chamber and a second cardiac signal in a second heart chamber.

US20130053714A1 describes a method for operating an implantable medical device which includes delivering a plurality of pacing pulses to an atria of a patient's heart and monitoring intrinsic atrial activity to detect intrinsic atrial contractions between one or more of the plurality of pacing pulses.

It is an object of the present invention to provide an implantable medical device being particularly appropriate for recognizing atrial undersensing.

This object is achieved with an implantable medical device having the features of claim 1. The present invention is defined by the appended claims.

The device of the present disclosure comprises a processor, a memory unit, a first detection unit configured to detect an atrial electric signal of a human or animal heart, and a second detection unit configured to detect a ventricular electric signal of the same heart.

In an aspect of the present invention, the memory unit comprises a computer-readable program that causes the processor to perform the step explained in the following when executed on the processor. The performed step comprises an evaluation of atrial events in an atrial electric signal detected by the first detection unit and/ or ventricular events in a ventricular electric signal detected by the second detection unit for recognizing a condition of the implantable medical device in which atrial events in the atrial electric signal are only insufficiently detected. This evaluation is carried out by applying at least one of the following criteria:
- a morphology of the detected atrial electric signals,
- a lacking stability of atrial events,
- an absence of atrial events over a first period of time,
- a detection of an amplitude of the detected atrial electric signals being lower than a predefined threshold value,
- an absence of atrial events while ventricular events are detected within the same time window,
- a comparison between atrial events sensed with a first sensing profile of the first detection unit on the one hand and atrial events sensed with a second sensing profile of the first detection unit on the other hand. In this context, the second sensing profile is more sensitive than the first sensing profile.

The basic concept underlying the presently claimed invention is a reliable detection of atrial undersensing, e.g., detection of an occurrence of atrial undersensing in such an incidence that appropriate countermeasures are to be taken in order to guarantee a safe detection of atrial signals. For this purpose, the claimed implantable medical device performs an automatic continuous evaluation of sensed signals, wherein optionally a linkage of individually sensed signals or obtained information is possible. In doing so, the implantable medical device can distinguish between a condition in which atrial undersensing is present and a condition in which no atrial undersensing is present.

According to the present invention, an atrial event is defined as an atrial activity detected in an electrical signal. For instance, such an electrical signal can be recorded with an electrode anchored in the atrium, or an electrode anchored in a different heart chamber (e.g. in the ventricle, the signal is then measured via a far-field measurement), or using floating electrodes which are located within the atrium without being anchored to the heart wall. A typical atrial event is a P-wave.

According to the present invention, a ventricular event is defined as a ventricular activity detected in an electrical signal. For instance, such an electrical signal can be recorded with an electrode anchored in the ventricle, or an electrode anchored in a different heart chamber (e.g. in the atrium, the signal is then measured via a far-field measurement). A typical ventricular event is an R-wave.

In an embodiment, the morphology of the detected atrial events is used as basis for determining whether or not an atrial undersensing is present. Such a morphologic evaluation can be done by comparing two patterns of atrial electric signals with each other. One possible method for such a morphologic evaluation is a trigger-based evaluation. For such trigger-based evaluation, the second detection unit generates ventricular triggers marking e.g. the end of the search interval for detection of an atrial event.

Afterwards, a signal section is extracted in the far-field channel of the obtained atrial electric signals around the time point of the trigger (e.g., between 200 ms, in particular 100 ms, in particular 90 ms, in particular 80 ms, in particular 70 ms, in particular 60 ms, in particular 50 ms, in particular 40 ms, in particular 30 ms, in particular 20 ms, in particular 10 ms before the generated trigger and 10 ms, in particular 20 ms, in particular 30 ms, in particular 40 ms, in particular 50 ms, in particular 60 ms, in particular 70 ms, in particular 80 ms, in particular 90 ms, in particular 100 ms after the generated trigger). According to a preferred embodiment, a signal section is extracted 200 ms before and 50 ms after the ventricular trigger, which would ensure to include AV-node-reentry tachycardia scenarios and a wide range of typical antegrade conducted 1: 1 rhythm scenarios (SVTs).

Subsequently, features are extracted from this signal section. Such an extraction can be done, e.g., by determining the peak-to-peak amplitude (calculating the maximum distance between the lowest and the highest voltage value); by calculating a quotient between the area under a peak of the electric atrial signals and the value of the maximum peak-to-peak amplitude; by generating a multi-dimensional vector in which each calculated feature forms an own dimension (jagged difference vector), as explained in detail in EP 2 353 644 A1.

Furthermore, the feature extraction as explained in the preceding paragraph is also carried out for the reference pattern.

Afterwards, a morphologic distance between the actually recorded atrial electric signal and the reference pattern can be calculated by determining the Euclidean distance between the vectors obtained by the feature extraction of the current atrial signal and of the reference pattern.

Finally, the scalar result of the preceding calculation is compared with a threshold value. If the scalar result is above the threshold value, a significant distance to the reference pattern is given so that no atrial event (P wave) is to be seen in the currently chosen atrial electric signal section (i.e., no peak in the atrial signal resulting from atrial activity). If, however, the result is below the threshold value, the similarities between the currently measured atrial electric signal section and the reference pattern are sufficiently high to recognize a P wave in the currently measured atrial signal.

Another method for comparing two patterns with each other is a correlation-based method. For such a method, longer blocks are excised out of the currently measured atrial electric signal. These longer blocks may have a duration of up to 20 seconds, e.g. 1 second to 20 seconds, in particular 2 seconds to 19 seconds, in particular 3 seconds to 18 seconds, in particular 4 seconds to 17 seconds, in particular 5 seconds to 16 seconds, in particular 6 seconds to 15 seconds, in particular 7 seconds to 14 seconds, in particular 8 seconds to 13 seconds, in particular 9 seconds to 12 seconds, in particular 10 seconds to 11 seconds. A P wave reference pattern is overlaid to these blocks by moving the reference pattern along these blocks. In doing so, correlation coefficients are continuously calculated. The local maximum of the correlation constitute potential P wave events in the currently measured atrial signal.

All local maxima above a threshold value are considered to be recognized atrial events (P waves). The amount of the latest confirmed P wave events is correlated with the block length in order to determine atrial tachycardia. E.g., if at least 10, in particular at least 12, in particular at least 14, in particular at least 16, in particular at least 18, in particular at least 20 P wave events are detected within a block length of, e.g., 10 seconds, atrial tachycardia is present.

It is also possible to train the implantable medical device to facilitate detection of P waves, i.e., to assign real atrial events to detected atrial electric signals. For such training purposes, the implantable medical device records at a (slow) sinus rhythm (less than 100 beats per minute (bpm), in particular less than 95 bpm, in particular less than 90 bpm, in particular less than 85 bpm, in particular less and 80 bpm, in particular less than 75 bpm, in particular less than 70 bpm) a sufficiently long signal section (bigger than 55 seconds, in particular bigger than 60 seconds, in particular bigger than 65 seconds, in particular bigger than 70 seconds, in particular bigger than 75 seconds, in particular bigger than 80 seconds, in particular bigger than 85 seconds, in particular bigger than 90 seconds). This signal section is then used to extract all atrial events as explained above. Subsequently, an average value of the individual features of the detected atrial electric signals is calculated.

In doing so, a reference pattern is generated which is characterized by an average value of, e.g., a maximum peak-to-peak amplitude, of a normalized area under the peak, and/or of a (jagged) difference vector.

It is then possible to automatically detect that the extracted regions from the recorded atrial electric signal (time frames around the automatically triggered atrial events) are considered to be P waves. Alternatively, medical doctor is needed to confirm that the extracted sections are to be considered as P waves.

Training of reference patterns for a correlation-based method can be done by extracting potential P wave signal sections from a recorded atrial electric signal by the implantable medical device. If a specific number of potential P wave events is detected (e.g., at least 20, in particular at least 25, in particular at least 30, in particular at least 35, in particular at least 40, in particular at least 45, in particular at least 50, in particular any interval that can be built up from the precedingly mentioned lower values), these potential P wave events can be presented to a medical doctor and can be manually confirmed to be P waves. This can likewise be done by machine learning process. This training - as the training in case of carrying out a trigger-based evaluation - needs to be done only once to build up a (patient-specific) reference pattern. Afterwards, the implantable medical device can always rely on this reference pattern for morphologically evaluating the obtained atrial electric signal.

For such a morphologic evaluation of the atrial signal, the detected pattern is compared with the P wave reference pattern, in particular with a patient-specific reference pattern. If a P wave is morphologically identified by such comparison, but if at the same time (with a predetermined temporal tolerance) no atrial event has been detected, atrial undersensing is present. Thus, in such a case atrial events can only be detected because of the morphologic evaluation of the atrial signal, but not from the atrial signal itself. This indicates that the atrial electric signals are only insufficiently detected.

According to an embodiment of the present invention, the reference pattern for morphologic evaluation is automatically adaptable. This can counteract to morphologic drifts of the electric signal, e.g. caused by a changes of the drug regime or dehydration of the patient. For instance, all signals used for adaptation of the reference pattern need to fulfil certain quality requirements. As examples, a minimum signal to noise ratio has to be fulfilled, or the signals shall not be clipped.

In an embodiment, the evaluation is carried out on the basis of a lacking stability of atrial events. A determination of the atrial stability can be realized by a comparison of the presently measured atrial events with an average value of a predetermined number of consecutive earlier atrial events. The predetermined number can be, e.g., 2, 3, 4, 5, 6, 7, 8, 9, or 10. If the comparison results in a deviation above a predetermined threshold value, the atrial rhythm can be considered as being instable. While lacking atrial stability can be the result of a specific atrial rhythm such as atrial fibrillation, lacking atrial stability can also be an indication of atrial undersensing, i.e., of an insufficient detection of atrial signals, although the atrial rhythm itself is stable.

In an embodiment, the evaluation is carried out on the basis of an absence of atrial events over a first period of time. Since atrial events are generally expected to occur regularly, an absence of atrial events is a strong indication of atrial undersensing. Since an absence of atrial events might also be the symptom of a severe cardiac dysfunction, it is important to distinguish atrial undersensing from a 'real', i.e. physiological absence of atrial events.

In an embodiment, the evaluation is carried out on the basis of a detection of an amplitude of the detected atrial electric signal that is lower than a predetermined threshold value. If amplitudes below such threshold value are detected, the probability of atrial undersensing is strongly increased since typically the atrial signals are to be expected to lie over specific values that can be individually defined.

In an embodiment, the evaluation is carried out on the basis of an absence of atrial events while ventricular electric signals are detected at the same time. Typically, it is expected to detect atrial events while a specific number of right ventricular events are detected. If no atrial events can be detected despite a ventricular activity, this is a strong indication of atrial undersensing.

In an embodiment, the evaluation is carried out on the basis of a comparison between atrial events sensed with a first sensing profile of the first detection unit and atrial events sensed with a second profile of the first detection unit. In this context, the second sensing profile is more sensitive than the first sensing profile. If a deviation between a sensing result achieved with the second sensing profile and a sensing result achieved with the first sensing profile is observed, this indicates that an increased sensitivity results in an increased number of detected atrial events. This, in turn, indicates that atrial undersensing is present when the first sensing profile is applied. In a variant of this embodiment, the additional atrial events detected with the second sensing profile are tested as to whether or not they can be integrated into a cardiac rhythm observed when applying the first sensing profile. If this is the case, it is very likely that the first sensing profile is not sensitive enough to detect all atrial events and that the higher sensitive second sensing profile also reveals those atrial events that are to be expected to occur assuming a regular cardiac rhythm.

In an embodiment, the first period of time during which an absence of atrial events is taken, in an embodiment, as basis for evaluating atrial undersensing is a time between 1 and 5 seconds, e.g., 1.5 seconds, 2 seconds, 2.5 seconds, 3 seconds, 3.5 seconds, 4 seconds, or 4.5 seconds.

In an embodiment, the at least one criterion comprises an absence of atrial events over a period of time in which 2 to 5, e.g., 3 or 4, ventricular events are detected. If, e.g., 3 ventricular events are detected but at the same time no atrial events are detected, this strongly indicates atrial undersensing since atrial events are expected to occur prior to the detected ventricular events as these ventricular events are triggered by prior atrial events.

In an embodiment, not only a single of the precedingly explained criteria is used for evaluating the atrial electric signals to assess atrial undersensing, but rather 2, 3, 4, 5 or 6 of these criteria are used together in order to increase the accuracy of the evaluation method.

In an embodiment, the evaluation is carried out on the basis of the following criteria: (lacking stability of the detected atrial events) and ((absence of atrial events over a first period of time) or (absence of atrial events during detection of ventricular events at the same time)). Thus, in this embodiment, the evaluation is carried out on the basis of two of the preceding the explained criteria, wherein one of these criteria is fixed and the other can be chosen from two specific criteria.

In another embodiment, the evaluation is carried out on the basis of the following criteria: ((no detected atrial event while at least 3 consecutive ventricular events are detected) and (lacking stability of atrial events)) or (no detected atrial event during the first period of time) or (detection of an amplitude of the detected atrial electric signal being lower than a predefined threshold value). Thus, in this embodiment, a single or two combined criteria need to be fulfilled to suspect atrial undersensing. In an embodiment, the evaluation is carried out on the basis of the precedingly explained criteria, however, without considering lacking stability of atrial events and without considering a detection of an amplitude of the detected atrial electric signal being lower than a predefined threshold value, i.e., only on the basis of the following criteria: ((no detected atrial event while at least 3 consecutive ventricular events are detected) and (lacking stability of atrial events)) or (no atrial event the first period of time).

In an embodiment, the evaluation is carried out on the basis of the following criteria: (no detected atrial event while at least 3 consecutive ventricular events are detected) or (detection of an amplitude of the detected atrial electric signal being lower than a predefined threshold value). Thus, in this embodiment, either one or the other criterion is sufficient to perform the evaluation of the detected atrial signal in order to assess whether or not atrial undersensing is present.

In an embodiment, the computer-readable program causes the processor to perform the following steps when executed on the processor: sending a signal to a home monitoring service center (HMSC) that serves for monitoring the health status of the patient carrying the implantable device. By such a signal, it is indicated in the HMSC that atrial undersensing has been detected and that countermeasures are necessary or are automatically taken. If a specific number of atrial undersensing events are detected, this indicates that there might be a general problem with the electrode used for sensing the atrial signals. In such a case, a reposition or a limitation of the floating radius of the electrode might be necessary.

In an embodiment, the computer-readable program causes the processor to perform the following step when executed on the processor: switching the implantable medical device from multi-chamber detection logic to single-chamber detection logic. In an embodiment, the multi-chamber detection logic is a two-chamber detection logic considering both atrial and ventricular signals for deciding whether or not a stimulation of the human or animal heart is necessary. The single-chamber detection logic is typically a ventricular detection logic that only uses ventricular signals to decide whether or not an external stimulation is necessary. If atrial undersensing has been detected, it is sensible to rely - at least for a certain period of time - no longer on the insufficiently detected atrial signals, but rather to rely only on ventricular signals in order to make a decision if stimulation of the heart to be treated is necessary.

In an embodiment, the computer-readable program causes the processor to perform the following step when executed on the processor: automatically adjusting at least one detection parameter of the first detection unit. Such a parameter of the first detection unit is typically a parameter of a detection algorithm applied by the first detection unit. By an adjustment of such parameter, the detection performance of the first detection unit can be optimized, e.g., with respect to weak atrial electric signals.

In an embodiment, the computer-readable program causes the processor to perform the following step when executed on the processor: automatically switching from a first sensing profile of the first detection unit to a second sensing profile of the first detection unit. In this context, the second sensing profile is more sensitive than the first sensing profile. By applying a different sensing profile having a higher sensitivity, it is possible to detect, e.g., weaker atrial electric signals with a higher reliability. In an embodiment, the second sensing profile guarantees that the sensitivity is, after detection of an atrial event, only reduced to a predefined value so that the sensitivity in the second sensing profile remains higher for subsequent atrial signals than in case of the first sensing profile. In an embodiment, the second sensing profile guarantees to increase the sensitivity, after having detected an atrial event, to a higher extent as the first sensing profile does. This facilitates the detection of any subsequent atrial events to be detected by application of the second sensing profile.

In an embodiment, the computer-readable program causes the processor to perform the following step when executed on the processor: electronically adding an atrial event to the detected atrial electric signals if a P wave is extracted during a morphological evaluation of a far-field potential detected by the first detection unit. Typically, such P wave extraction is carried out by recognizing a congruency between a reference signal comprising a P wave and the far-field potential detected by the first detection unit. By such an electronic addition of atrial signals to the detected signal, missing atrial events can be compensated so that a complete atrial signal results that can be further evaluated.

In an embodiment, care is taken that the implantable medical device does not remain in a specific atrial undersensing mode for an indefinite time. Rather, measures are taken in order to reset the implantable medical device to normal mode detection if no further atrial undersensing is to be expected or can be detected. For this purpose, in an embodiment, the computer-readable program causes the processor to perform the following step when executed on the processor: automatically switching back from an operational mode adopted by the implantable medical device after having recognized insufficient detection of atrial events (atrial undersensing mode) to a regular operational mode (normal mode) if a tachycardic rhythm of the treated heart has been terminated or if no insufficient detection of atrial events has been recognized over a second period of time. In an embodiment, the second period of time is a time between 10 minutes and 60 minutes, in particular between 20 minutes and 50 minutes, in particular between 30 minutes and 40 minutes.

The present disclosure also relates to a method for recognizing a condition of an implantable medical device according to the preceding explanations. The condition to be recognized is characterized by an insufficient detection of atrial events. In this context, the method comprises the step explained in the following.

The performed step comprises an evaluation of atrial events in an atrial electric signal detected by a first detection unit and/or ventricular events in a ventricular electric signal detected by a second detection unit of the implantable medical device for recognizing a condition of the implantable medical device in which atrial events are only insufficiently detected. This evaluation is carried out on the basis of at least one of the following criteria:
- a morphology of the detected atrial electric signals,
- a lacking stability of atrial events,
- an absence of atrial events over a first period of time,
- a detection of an amplitude of the detected atrial electric signals being lower than a predefined threshold value,
- an absence of atrial events while ventricular events are detected within the same time window, a comparison between atrial events sensed with a first sensing profile of the first detection unit on the one hand and atrial events sensed with a second sensing profile of the first detection unit on the other hand. In this context, the second sensing profile is more sensitive than the first sensing profile.

The present disclosure also relates to computer program product comprising computer-readable code that causes the processor to perform the step explained in the following when executed on the processor.

The performed step comprises an evaluation of atrial events in an atrial electric signal detected by a first detection unit and/or ventricular events in a ventricular electric signal detected by a second detection unit of an implantable medical device for recognizing a condition of the implantable medical device in which atrial events are only insufficiently detected. This evaluation is carried out on the basis of at least one of the following criteria:
- a morphology of the detected atrial electric signals,
- a lacking stability of atrial events,
- an absence of atrial events over a first period of time,
- a detection of an amplitude of the detected atrial electric signals being lower than a predefined threshold value,
- an absence of atrial events while ventricular events are detected within the same time window,
- a comparison between atrial events sensed with a first sensing profile of the first detection unit on the one hand and atrial events sensed with a second sensing profile of the first detection unit on the other hand. In this context, the second sensing profile is more sensitive than the first sensing profile.

The present disclosure also relates to an exemplary, non-claimed method of treatment of human or animal patient in need of such treatment by means of an implantable medical device according to the preceding explanations. This implantable medical device comprises a processor, a memory unit, a first detection unit configured to detect an atrial electric signal of a human or animal heart, a second detection unit configured to detect a ventricular electric signal of the same heart and a stimulation unit configured to stimulate a cardiac region of the same heart. In this context, the method comprises the steps explained the following.

In a first step atrial events in the atrial electric signal detected by the first detection unit and/or ventricular events in the ventricular electric signal detected by the second detection unit of the implantable medical device are evaluated for recognizing a condition of the implantable medical device in which atrial events are only insufficiently detected. This evaluation is carried out on the basis of at least one of the following criteria:
- a morphology of the detected atrial electric signals,
- a lacking stability of atrial events,
- an absence of atrial events over a first period of time,
- a detection of an amplitude of the detected atrial electric signals being lower than a predefined threshold value,
- an absence of atrial events while ventricular events are detected within the same time window,
- a comparison between atrial events sensed with a first sensing profile of the first detection unit on the one hand and atrial events sensed with a second sensing profile of the first detection unit on the other hand. In this context, the second sensing profile is more sensitive than the first sensing profile.

Subsequently, a detection mode of the first detection unit is automatically adjusted if a condition of the implantable medical device has been recognized in which atrial events are only insufficiently detected (atrial undersensing mode).

Subsequently, a cardiac region of the human or animal heart is stimulated by applying a stimulation pulse with the stimulation unit if such stimulation is deemed to be necessary upon an evaluation of the cardiac rhythm of the human or animal heart in the adjusted detection mode.

The present invention is defined by the appended claims.

## Claims

1. Implantable medical device, comprising a processor, a memory unit, a first detection unit configured to detect an atrial electric signal of a human or animal heart, and a second detection unit configured to detect a ventricular electric signal of the same heart, wherein the memory unit comprises a computer-readable program that causes the processor to perform the following step when executed on the processor:
evaluation of atrial events in the atrial electric signal detected by the first detection unit and/or ventricular events in the ventricular electric signal detected by the second detection unit for recognizing a condition of the implantable medical device in which atrial events in the atrial electric signal are detected only insufficiently, wherein the evaluation is carried out on the basis of at least one of the following criteria:
i) morphology of the detected atrial electric signal,
ii) lacking stability of atrial events,
iii) absence of atrial events over a first period of time,
iv) detection of an amplitude of the detected atrial electric signal being lower than a predefined threshold value,
v) absence of atrial events during detection of ventricular events at the same time,
vi) comparison of atrial events sensed with a first sensing profile of the first detection unit and atrial events sensed with a second sensing profile of the first detection unit, the second sensing profile being more sensitive than the first sensing profile,
**characterized**
**in that** the evaluation is carried out on the basis of the following criteria:
i) stability of the detected atrial events and ii) absence of atrial events over a first period of time or absence of atrial events during detection of ventricular events at the same time.

2. Implantable medical device according to claim 1, **characterized in that** the first period of time is a time between 1 and 5 seconds.

3. Implantable medical device according to claim 1 or 2, **characterized in** the at least one criterion comprises an absence of atrial events over a period of time in which 2 to 5 ventricular events are detected.

4. Implantable medical device according to any of the preceding claims, **characterized in that** the evaluation is carried out on the basis of the following criteria: i) no detected atrial event while at least 3 ventricular events are detected and lacking stability of atrial events or ii) no detected P wave during the first period of time or iii) detection of an amplitude of the detected atrial electric signal being lower than a predefined threshold value.

5. Implantable medical device according to any of the preceding claims, **characterized in that** the evaluation is carried out on the basis of the following criteria: i) no detected atrial event while at least 3 ventricular events are detected or ii) detection of an amplitude of the detected atrial electric signal being lower than a predefined threshold value.

6. Implantable medical device according to any of the preceding claims, **characterized in that** the computer-readable program causes the processor to perform the following step when executed on the processor: sending a signal to a home monitoring service center that serves for monitoring a health status of a patient carrying the implantable device.

7. Implantable medical device according to any of the preceding claims, **characterized in that** the computer-readable program causes the processor to perform the following step when executed on the processor: switching the implantable medical device from a multi-chamber detection logic to a one-chamber detection logic.

8. Implantable medical device according to any of the preceding claims, **characterized in that** the computer-readable program causes the processor to perform the following step when executed on the processor: automatically adjusting at least one detection parameter of the first detection unit.

9. Implantable medical device according to any of the preceding claims, **characterized in that** the computer-readable program causes the processor to perform the following step when executed on the processor: automatically switching from a first sensing profile of the first detection unit to a second sensing profile of the first detection unit, the second sensing profile being more sensitive than the first sensing profile.

10. Implantable medical device according to any of the preceding claims, **characterized in that** the computer-readable program causes the processor to perform the following step when executed on the processor: electronically adding an atrial event to the detected atrial electric signals if a P wave is extracted in course of a morphological evaluation of a far-field potential detected by the first detection unit.

11. Implantable medical device according to any of the preceding claims, **characterized in that** the computer-readable program causes the processor to perform the following step when executed on the processor: automatically switching back from an operational mode adopted by the implantable medical device after having recognized an insufficient detection of atrial electric signals to a regular operational mode if a tachycardic rhythm has been terminated or if no insufficient detection of atrial electric signals has been recognized over a second period of time.

12. Method for recognizing a condition of an implantable medical device according to any of the preceding claims in which condition atrial electric signals are detected only insufficiently, the method comprising the following step:
evaluation of atrial events in the atrial electric signal detected by a first detection unit and/or ventricular events in the ventricular electric signal detected by the second detection unit of the implantable medical device for recognizing a condition of the implantable medical device in which atrial events in the atrial electric signal are detected only insufficiently, wherein the evaluation is carried out on the basis of at least one of the following criteria:
i) morphology of the detected atrial electric signal,
ii) lacking stability of atrial events,
iii) absence of atrial events over a first period of time,
iv) detection of an amplitude of the detected atrial electric signal being lower than a predefined threshold value,
v) absence of atrial events during detection of ventricular events with a second detection unit of the implantable medical device at the same time,
vi) comparison of atrial events sensed with a first sensing profile of the first detection unit and atrial events sensed with a second sensing profile of the first detection unit, the second sensing profile being more sensitive than the first sensing profile,
wherein the evaluation is carried out on the basis of the following criteria:
i) stability of the detected atrial events and ii) absence of atrial events over a first period of time or absence of atrial events during detection of ventricular events at the same time.

13. Computer program product comprising computer-readable code that causes the processor of the implantable medical device of any of claims 1-11 to perform the following step when executed on the processor
evaluation of atrial events in the atrial electric signal detected by a first detection unit and/or ventricular events in the ventricular electric signal detected by the second detection unit of an implantable medical device for recognizing a condition of the implantable medical device in which atrial events in the atrial electric signal are detected only insufficiently, wherein the evaluation is carried out on the basis of at least one of the following criteria:
i) morphology of the detected atrial electric signal,
ii) lacking stability of atrial events,
iii) absence of atrial events over a first period of time,
iv) detection of an amplitude of the detected atrial electric signal being lower than a predefined threshold value,
v) absence of atrial events during detection of ventricular events at the same time,
vi) comparison of atrial electric signals sensed with a first sensing profile of the first detection unit and atrial events sensed with a second sensing profile of the first detection unit, the second sensing profile being more sensitive than the first sensing profile,
wherein the evaluation is carried out on the basis of the following criteria:
i) stability of the detected atrial events and ii) absence of atrial events over a first period of time or absence of atrial events during detection of ventricular events at the same time.

## Patentansprüche

1. Implantierbares Medizinprodukt, einen Prozessor, eine Speichereinheit, eine erste Detektionseinheit, die dafür konfiguriert ist, ein atriales elektrisches Signal eines menschlichen oder tierischen Herzens zu detektieren, und eine zweite Detektionseinheit, die dafür konfiguriert ist, ein ventrikuläres elektrisches Signal desselben Herzens zu detektieren, umfassend, wobei die Speichereinheit ein computerlesbares Programm umfasst, das den Prozessor veranlasst, bei Ausführung auf dem Prozessor den folgenden Schritt durchzuführen:
Auswertung atrialer Ereignisse in dem atrialen elektrischen Signal, das von der ersten Detektionseinheit detektiert wird, und/oder ventrikulärer Ereignisse in dem ventrikulären elektrischen Signal, das von der zweiten Detektionseinheit detektiert wird, um eine Bedingung des implantierbaren Medizinprodukts zu erkennen, bei der atriale Ereignisse in dem atrialen elektrischen Signal nur unzureichend erkannt werden, wobei die Auswertung auf der Basis von mindestens einem der folgenden Kriterien durchgeführt wird:
i) Morphologie des detektierten atrialen elektrischen Signals,
ii) mangelnde Stabilität atrialer Ereignisse,
iii) keine atrialen Ereignisse über einen ersten Zeitraum,
iv) Detektion einer Amplitude des detektierten atrialen elektrischen Signals, die niedriger ist als ein vordefinierter Schwellenwert,
v) keine atrialen Ereignisse während der Detektion ventrikulärer Ereignisse zur selben Zeit,
vi) Vergleich atrialer Ereignisse, die mit einem ersten Sensing-Profil der ersten Detektionseinheit erfasst werden, und atrialer Ereignisse, die mit einem zweiten Sensing-Profil der ersten Detektionseinheit erfasst werden, wobei das zweite Sensing-Profil empfindlicher ist als das erste Sensing-Profil,
**dadurch gekennzeichnet,**
**dass** die Auswertung auf der Basis der folgenden Kriterien durchgeführt wird: i) Stabilität der detektierten atrialen Ereignisse, und ii) keine atrialen Ereignisse über einen ersten Zeitraum oder keine atrialen Ereignisse während der Detektion ventrikulärer Ereignisse zur selben Zeit.

2. Implantierbares Medizinprodukt nach Anspruch 1, **dadurch gekennzeichnet, dass** der erste Zeitraum eine Zeit zwischen 1 und 5 Sekunden ist.

3. Implantierbares Medizinprodukt nach Anspruch 1 oder 2, **dadurch gekennzeichnet, dass** das mindestens eine Kriterium eine Abwesenheit atrialer Ereignisse über einen Zeitraum, in dem 2 bis 5 ventrikuläre Ereignisse detektiert werden, umfasst.

4. Implantierbares Medizinprodukt nach einem der vorstehenden Ansprüche, **dadurch gekennzeichnet, dass** die Auswertung auf der Basis der folgenden Kriterien erfolgt: i) kein detektiertes atriales Ereignis, während mindestens 3 ventrikuläre Ereignisse detektiert werden, und mangelnde Stabilität atrialer Ereignisse, oder ii) keine detektierte P-Welle in dem ersten Zeitraum oder iii) Detektion einer Amplitude des detektierten atrialen elektrischen Signals, die niedriger ist als ein vordefinierter Schwellenwert.

5. Implantierbares Medizinprodukt nach einem der vorstehenden Ansprüche, **dadurch gekennzeichnet, dass** die Auswertung auf der Basis der folgenden Kriterien erfolgt: i) kein detektiertes atriales Ereignis, während mindestens 3 ventrikuläre Ereignisse detektiert werden, oder ii) Detektion einer Amplitude des detektierten atrialen elektrischen Signals, die niedriger ist als ein vordefinierter Schwellenwert.

6. Implantierbares Medizinprodukt nach einem der vorstehenden Ansprüche, **dadurch gekennzeichnet, dass** das computerlesbare Programm den Prozessor veranlasst, bei Ausführung auf dem Prozessor den folgenden Schritt durchzuführen: Senden eines Signals an ein Home Monitoring Service Center, das dazu dient, einen Gesundheitszustand eines Patienten, der das implantierbare Produkt trägt, zu überwachen.

7. Implantierbares Medizinprodukt nach einem der vorstehenden Ansprüche, **dadurch gekennzeichnet, dass** das computerlesbare Programm den Prozessor veranlasst, bei Ausführung auf dem Prozessor den folgenden Schritt durchzuführen: Umschalten des implantierbaren Medizinprodukts von einer Mehrkammer-Detektionslogik zu einer Ein-Kammer-Detektionslogik.

8. Implantierbares Medizinprodukt nach einem der vorstehenden Ansprüche, **dadurch gekennzeichnet, dass** das computerlesbare Programm den Prozessor veranlasst, bei Ausführung auf dem Prozessor den folgenden Schritt durchzuführen: Automatisches Anpassen von mindestens einem Detektionsparameter der ersten Detektionseinheit.

9. Implantierbares Medizinprodukt nach einem der vorstehenden Ansprüche, **dadurch gekennzeichnet, dass** das computerlesbare Programm den Prozessor veranlasst, bei Ausführung auf dem Prozessor den folgenden Schritt durchzuführen: Automatisches Umschalten von einem ersten Sensing-Profil der ersten Detektionseinheit zu einem zweiten Sensing-Profil der ersten Detektionseinheit, wobei das zweite Sensing-Profil empfindlicher als das erste Sensing-Profil ist.

10. Implantierbares Medizinprodukt nach einem der vorstehenden Ansprüche, **dadurch gekennzeichnet, dass** das computerlesbare Programm den Prozessor veranlasst, bei Ausführung auf dem Prozessor den folgenden Schritt durchzuführen: Elektronisches Hinzufügen eines atrialen Ereignisses zu den detektierten atrialen elektrischen Signalen, wenn eine P-Welle im Zuge einer morphologischen Auswertung eines von der ersten Detektionseinheit detektierten Fernfeldpotentials extrahiert wird.

11. Implantierbares Medizinprodukt nach einem der vorstehenden Ansprüche, **dadurch gekennzeichnet, dass** das computerlesbare Programm den Prozessor veranlasst, bei Ausführung auf dem Prozessor den folgenden Schritt durchzuführen: Automatisches Zurückschalten aus einem Betriebsmodus, in den das implantierbare Medizinprodukt gegangen ist, nachdem eine unzureichende Detektion atrialer elektrischer Signale erkannt wurde, in einen regulären Betriebsmodus, wenn ein tachykarder Rhythmus beendet worden ist oder wenn über einen zweiten Zeitraum keine unzureichende Detektion atrialer elektrischer Signale erkannt wurde.

12. Verfahren zum Erkennen einer Bedingung eines implantierbaren Medizinprodukts nach einem der vorstehenden Ansprüche, wobei unter dieser Bedingung atriale elektrische Signale nur unzureichend detektiert werden, wobei das Verfahren den folgenden Schritt umfasst:
Auswertung atrialer Ereignisse in dem atrialen elektrischen Signal, das von einer ersten Detektionseinheit detektiert wird, und/oder ventrikulärer Ereignisse in dem ventrikulären elektrischen Signal, das von der zweiten Detektionseinheit des implantierbaren Medizinprodukts detektiert wird, um eine Bedingung des implantierbaren Medizinprodukts zu erkennen, bei der atriale Ereignisse in dem atrialen elektrischen Signal nur unzureichend erkannt werden, wobei die Auswertung auf der Basis von mindestens einem der folgenden Kriterien durchgeführt wird:
i) Morphologie des detektierten atrialen elektrischen Signals,
ii) mangelnde Stabilität atrialer Ereignisse,
iii) keine atrialen Ereignisse über einen ersten Zeitraum,
iv) Detektion einer Amplitude des detektierten atrialen elektrischen Signals, die niedriger ist als ein vordefinierter Schwellenwert,
v) keine atrialen Ereignisse bei der Detektion ventrikulärer Ereignisse mit einer zweiten Detektionseinheit des implantierbaren Medizinprodukts zur selben Zeit,
vi) Vergleich atrialer Ereignisse, die mit einem ersten Sensing-Profil der ersten Detektionseinheit erfasst werden, und atrialer Ereignisse, die mit einem zweiten Sensing-Profil der ersten Detektionseinheit erfasst werden, wobei das zweite Sensing-Profil empfindlicher ist als das erste Sensing-Profil,
wobei die Auswertung auf der Basis der folgenden Kriterien erfolgt i) Stabilität der detektierten atrialen Ereignisse, und ii) keine atrialen Ereignisse über einen ersten Zeitraum oder keine atrialen Ereignisse bei der Detektion ventrikulärer Ereignisse zur selben Zeit.

13. Computerprogrammprodukt, einen computerlesbaren Code umfassend, der den Prozessor des implantierbaren Medizinprodukts nach einem der Ansprüche 1-11 veranlasst, bei Ausführung auf dem Prozessor den folgenden Schritt durchzuführen:
Auswertung atrialer Ereignisse in dem atrialen elektrischen Signal, das von einer ersten Detektionseinheit detektiert wird, und/oder ventrikulärer Ereignisse in dem ventrikulären elektrischen Signal, das von der zweiten Detektionseinheit eines implantierbaren Medizinprodukts detektiert wird, um eine Bedingung des implantierbaren Medizinprodukts zu erkennen, bei der atriale Ereignisse in dem atrialen elektrischen Signal nur unzureichend erkannt werden, wobei die Auswertung auf der Basis von mindestens einem der folgenden Kriterien durchgeführt wird:
i) Morphologie des detektierten atrialen elektrischen Signals,
ii) mangelnde Stabilität atrialer Ereignisse,
iii) keine atrialen Ereignisse über einen ersten Zeitraum,
iv) Detektion einer Amplitude des detektierten atrialen elektrischen Signals, die niedriger ist als ein vordefinierter Schwellenwert,
v) keine atrialen Ereignisse bei der Detektion ventrikulärer Ereignisse zur selben Zeit,
vi) Vergleich atrialer Ereignisse, die mit einem ersten Sensing-Profil der ersten Detektionseinheit erfasst werden, und atrialer Ereignisse, die mit einem zweiten Sensing-Profil der ersten Detektionseinheit erfasst werden, wobei das zweite Sensing-Profil empfindlicher ist als das erste Sensing-Profil,
wobei die Auswertung auf der Basis der folgenden Kriterien erfolgt:
i) Stabilität der detektierten atrialen Ereignisse, und ii) keine atrialen Ereignisse über einen ersten Zeitraum oder keine atrialen Ereignisse bei der Detektion ventrikulärer Ereignisse zur selben Zeit.

## Revendications

1. Dispositif médical implantable, comprenant un processeur, une unité de mémoire, une première unité de détection configurée pour détecter un signal électrique auriculaire d'un cœur humain ou animal, et une seconde unité de détection configurée pour détecter un signal électrique ventriculaire du même cœur, dans lequel l'unité de mémoire comprend un programme lisible par ordinateur qui amène le processeur à réaliser l'étape suivante lorsqu'il est exécuté sur le processeur : évaluation d'événements auriculaires dans le signal électrique auriculaire détecté par la première unité de détection et/ou d'événements ventriculaires dans le signal électrique ventriculaire détecté par la seconde unité de détection pour reconnaître un état du dispositif médical implantable dans lequel les événements auriculaires dans le signal électrique auriculaire sont détectés uniquement de façon insuffisante, dans lequel l'évaluation est mise en œuvre sur la base d'au moins l'un des critères suivants :
i) morphologie du signal électrique auriculaire détecté,
ii) manque de stabilité des événements auriculaires,
iii) absence d'événements auriculaires sur une première période,
iv) détection d'une amplitude du signal électrique auriculaire détecté comme étant inférieure à une valeur de seuil prédéfinie,
v) absence d'événements auriculaires pendant la détection d'événements ventriculaires simultanée,
vi) comparaison d'événements auriculaires détectés avec un premier profil de détection de la première unité de détection et d'événements auriculaires détectés avec un second profil de détection de la première unité de détection, le second profil de détection étant plus sensible que le premier profil de détection,
**caractérisé**
**en ce que** l'évaluation est mise en œuvre sur la base des critères suivants : i) stabilité des événements auriculaires détectés et ii) absence d'événements auriculaires sur une première période ou absence d'événements auriculaires pendant la détection d'événements ventriculaires simultanée.

2. Dispositif médical implantable selon la revendication 1, **caractérisé en ce que** la première période est une durée comprise entre 1 et 5 secondes.

3. Dispositif médical implantable selon la revendication 1 ou 2, **caractérisé en ce qu'**au moins un critère comprend une absence d'événements auriculaires sur une période dans laquelle 2 à 5 événements ventriculaires sont détectés.

4. Dispositif médical implantable selon l'une quelconque des revendications précédentes, **caractérisé en ce que** l'évaluation est mise en œuvre sur la base des critères suivants : i) aucun événement auriculaire détecté tandis qu'au moins 3 événements ventriculaires sont détectés et manque de stabilité des événements auriculaires ou ii) aucune onde P détectée pendant la première période ou iii) détection d'une amplitude du signal électrique auriculaire détecté comme étant inférieure à une valeur de seuil prédéfinie.

5. Dispositif médical implantable selon l'une quelconque des revendications précédentes, **caractérisé en ce que** l'évaluation est mise en œuvre sur la base des critères suivants : i) aucun événement auriculaire détecté tandis qu'au moins 3 événements ventriculaires sont détectés ou ii) détection d'une amplitude du signal électrique auriculaire détecté comme étant inférieure à une valeur de seuil prédéfinie.

6. Dispositif médical implantable selon l'une quelconque des revendications précédentes, **caractérisé en ce que** le programme lisible par ordinateur amène le processeur à réaliser l'étape suivante lorsqu'il est exécuté sur le processeur : envoyer un signal à un centre de services de surveillance à domicile qui sert à surveiller un statut de santé d'un patient portant le dispositif implantable.

7. Dispositif médical implantable selon l'une quelconque des revendications précédentes, **caractérisé en ce que** le programme lisible par ordinateur amène le processeur à réaliser l'étape suivante lorsqu'il est exécuté sur le processeur : commuter le dispositif médical implantable depuis une logique de détection multi-chambres vers une logique de détection à une chambre.

8. Dispositif médical implantable selon l'une quelconque des revendications précédentes, **caractérisé en ce que** le programme lisible par ordinateur amène le processeur à réaliser l'étape suivante lorsqu'il est exécuté sur le processeur : ajuster automatiquement au moins un paramètre de détection de la première unité de détection.

9. Dispositif médical implantable selon l'une quelconque des revendications précédentes, **caractérisé en ce que** le programme lisible par ordinateur amène le processeur à réaliser l'étape suivante lorsqu'il est exécuté sur le processeur : commuter automatiquement depuis un premier profil de détection de la première unité de détection vers un second profil de détection de la première unité de détection, le second profil de détection étant plus sensible que le premier profil de détection.

10. Dispositif médical implantable selon l'une quelconque des revendications précédentes, **caractérisé en ce que** le programme lisible par ordinateur amène le processeur à réaliser l'étape suivante lorsqu'il est exécuté sur le processeur : ajouter électroniquement un événement auriculaire aux signaux électriques auriculaires détectés si une onde P est extraite au cours d'une évaluation morphologique d'un potentiel de champ lointain détecté par la première unité de détection.

11. Dispositif médical implantable selon l'une quelconque des revendications précédentes, **caractérisé en ce que** le programme lisible par ordinateur amène le processeur à réaliser l'étape suivante lorsqu'il est exécuté sur le processeur : commuter automatiquement en retour depuis un mode de fonctionnement adopté par le dispositif médical implantable après avoir reconnu une détection insuffisante de signaux électriques auriculaires vers un mode de fonctionnement courant si un rythme tachycardique s'est terminé ou si aucune détection insuffisante de signaux électriques auriculaires n'a été reconnue sur une seconde période.

12. Procédé de reconnaissance d'un état d'un dispositif médical implantable selon l'une quelconque des revendications précédentes, état dans lequel les signaux électriques auriculaires sont détectés uniquement de manière insuffisante, le procédé comprenant l'étape suivante :
évaluation d'événements auriculaires dans le signal électrique auriculaire détecté par une première unité de détection et/ou d'événements ventriculaires dans le signal électrique ventriculaire détecté par la seconde unité de détection du dispositif médical implantable pour reconnaître un état du dispositif médical implantable dans lequel les événements auriculaires dans le signal électrique auriculaire sont détectés uniquement de façon insuffisante, dans lequel l'évaluation est mise en œuvre sur la base d'au moins l'un des critères suivants :
i) morphologie du signal électrique auriculaire détecté,
ii) manque de stabilité des événements auriculaires,
iii) absence d'événements auriculaires sur une première période,
iv) détection d'une amplitude du signal électrique auriculaire détecté comme étant inférieure à une valeur de seuil prédéfinie,
v) absence d'événements auriculaires pendant la détection d'événements ventriculaires avec une seconde unité de détection du dispositif médical implantable simultanée,
vi) comparaison d'événements auriculaires détectés avec un premier profil de détection de la première unité de détection et d'événements auriculaires détectés avec un second profil de détection de la première unité de détection, le second profil de détection étant plus sensible que le premier profil de détection,
dans lequel l'évaluation est mise en œuvre sur la base des critères suivants :
i) stabilité des événements auriculaires détectés et ii) absence d'événements auriculaires sur une première période ou absence d'événements auriculaires pendant la détection d'événements ventriculaires simultanée.

13. Produit de programme informatique comprenant un code lisible par ordinateur qui amène le processeur du dispositif médical implantable selon l'une quelconque des revendications 1 à 11 à réaliser l'étape suivante lorsqu'il est exécuté sur le processeur :
évaluation d'événements auriculaires dans le signal électrique auriculaire détecté par une première unité de détection et/ou d'événements ventriculaires dans le signal électrique ventriculaire détecté par la seconde unité de détection d'un dispositif médical implantable pour reconnaître un état du dispositif médical implantable dans lequel les événements auriculaires dans le signal électrique auriculaire sont détectés uniquement de façon insuffisante, dans lequel l'évaluation est mise en œuvre sur la base d'au moins l'un des critères suivants :
i) morphologie du signal électrique auriculaire détecté,
ii) manque de stabilité des événements auriculaires,
iii) absence d'événements auriculaires sur une première période,
iv) détection d'une amplitude du signal électrique auriculaire détecté comme étant inférieure à une valeur de seuil prédéfinie,
v) absence d'événements auriculaires pendant la détection d'événements ventriculaires simultanée,
vi) comparaison de signaux électriques auriculaires détectés avec un premier profil de détection de la première unité de détection et d'événements auriculaires détectés avec un second profil de détection de la première unité de détection, le second profil de détection étant plus sensible que le premier profil de détection,
dans lequel l'évaluation est mise en œuvre sur la base des critères suivants :
i) stabilité des événements auriculaires détectés et ii) absence d'événements auriculaires sur une première période de temps ou absence d'événements auriculaires pendant la détection d'événements ventriculaires simultanée.
